# EUROPÄISCHE PATENTANMELDUNG

(11) **EP 2 371 419 A2**
(43) Veröffentlichungstag der Anmeldung: **05.10.2011**
(21) Anmeldenummer: 11156794.7
(22) Anmeldetag: 25.03.2011
(51) Int. Cl.: A61N 1/375

(54) **Elektrische Durchführung eines Kondensators für medizinische Implantate sowie Verfahren zur Herstellung und Verwendung einer solchen**

(30) Priorität: 29.03.2010 US 318403 P
(71) Anmelder: BIOTRONIK SE & Co. KG, 12359 Berlin (DE)
(72) Erfinder: Teske, Josef, 96103, Hallstadt (DE)
(74) Vertreter: Lindner-Vogt, Karin L.

(57) **Zusammenfassung**

Durchführung eines Elektrolytkondensators, insbesondere in einem medizinelektronischen Implantat, mit einem Anschlussstift, der mindestens im Inneren des Elektrolytkondensators einen weichlötbaren Abschnitt aufweist, einem den Anschlussstift umgebenden Aluminium-Flansch und einem den Anschlussstift gegenüber dem Aluminium-Flansch hermetisch abdichtenden Glaslot-Pfropf.

## Beschreibung

Die Erfindung betrifft eine sogenannte Durchführung (den Anschlussstift-Bereich) eines Elektrolyt-, Tantal-, Niob- oder sonstigen Kondensators. Sie betrifft des Weiteren ein Verfahren zur Herstellung und Verwendung einer solchen Durchführung sowie einen Kondensator mit einer solchen Durchführung.

Kondensatoren, wie Elektrolyt-, Tantal- oder Niobkondensatoren zum Einsatz in medizinelektronischen Implantaten, etwa Defibrillatoren, sind in verschiedenen Ausführungen bekannt und im praktischen Einsatz. Aufbauten auf Basis von Al-Folien für Hochspannungsanwendungen, sogenannte Al-HV-Kondensatoren, sind in der Unterhaltungselektronik handelsüblich. Sie besitzen eine Abdichtung aus gummiartigen Stoffen oder Kunststoffen, die allerdings ein (sehr langsames) Entweichen von Elektrolytbestandteilen aus dem Kondensatorinneren nach außen nicht verhindert. Dadurch bedingt sind eine eingeschränkte Lebensdauer und Zuverlässigkeit und die Gefahr einer Kontamination des Inneren von Geräten, in denen sie eingebaut sind, also speziell der besagten Implantate. Bekannt sind auch Aufbauten auf Tantal- oder Niob-Basis, sogenannte Ta- oder Nb-HV-Kondensatoren, die aber im Vergleich zu Al-HV-Kondensatoren wesentlich schwerer sind und deren Herstellungstechnologie schwierig zu beherrschen ist.

Der Erfindung liegt die Aufgabe zugrunde, einen verbesserten Aufbau für Elektrolytkondensatoren vom Al-HV-Typ sowie auch für die anderen genannten Ta-, Nb- oder sonstigen Kondensatortypen anzugeben, der diesen insbesondere eine verbesserte Zuverlässigkeit, längere Lebensdauer und verbesserte Brauchbarkeit in medizinelektronischen Implantaten verleiht.

Diese Aufgabe wird gemäß ihrem Vorrichtungsaspekt durch eine Durchführung mit den Merkmalen des Anspruchs 1 und gemäß relativ unabhängigen Verfahrensaspekten durch ein Verfahren mit den Merkmalen des Anspruchs 14 bzw. 15 gelöst. Zweckmäßige Fortbildungen des Erfindungsgedankens sind Gegenstand der abhängigen Ansprüche.

Die Erfindung schließt den wesentlichen Gedanken ein, die gattungsgemäße Durchführung hermetisch dicht auszuführen, wobei die Durchführung das Kondensatorinnere gegenüber dem Kondensatoräußeren hermetisch dicht voneinander trennt. Sie schließt weiter den Gedanken des Vorsehens eines den Anschlussstift gegenüber dem Aluminium-Flansch elektrisch isolierenden Glaslot-Pfropfes ein. Der Anschlussstift ist hierbei mindestens an einem im Inneren des Kondensators liegenden Abschnitt mit dem Kondensatorinneren kompatibel ausgeführt. Im Zusammenhang mit der Erfindung ist von Bedeutung, dass im Inneren des Kondensators ausschließlich AI-Oberflächen und Isolator-Oberflächen vorliegen, aus denen keine Fremdionen in den Elektrolyten abgegeben werden, die dessen Selbstentladung unzulässig erhöhen und/oder seine Hochspannungsfestigkeit beeinträchtigen könnten. Die gleichen Maßnahmen gelten sinngemäß für Ta-, Nb- oder sonstige Kondensatortypen.

Eine zweckmäßige Ausführung der Erfindung sieht vor, dass der Anschlussstift in dem Bereich seiner Länge, in dem er in Kontakt mit dem Glaslot-Pfropf steht, mindestens einen Kern aus einem Material aufweist, das annähernd den gleichen oder einen kleineren thermischen Ausdehnungskoeffizienten wie das Glaslot besitzt. Hierdurch lassen sich unzulässig hohe mechanische Zugspannungen im Glaslot während des Herstellens und des Einsatzes der gefertigten Durchführung grundsätzlich vermeiden. Speziell kann der Anschlussstift oder sein Kern in dem Bereich seiner Länge, in dem er in Kontakt mit dem Glaslot-Pfropf steht, aus Pt, Pt/Ir, FeNi, FeNiCo, FeCr, Nb, Ta, Mo, W, Cr, FeCr, V, Ti und weiteren Metallen oder deren Legierungen bestehen.

In einer weiteren Ausführung der Erfindung ist der für die Elektrochemie wichtiger Abschnitt des Anschlussstifts im Inneren des Elektrolytkondensators ein Aluminiumstift, ein Aluminiumblech oder eine Aluminiumfolie. Hierbei ist insbesondere der Aluminiumstift, das Aluminiumblech oder die Aluminiumfolie an einen Abschnitt des Anschlussstifts aus einem anderen Material elektrisch leitend angefügt, insbesondere angelötet, angeschweißt, angesteckt, angecrimpt oder elektrisch leitfähig angeklebt. Aluminium als innenseitiges Material des Anschlussstifts ist vollständig kompatibel mit dem übrigen Aufbau speziell eines Al-HV-Kondensators und frei von störenden Ionenaustritteffekten. Diese Vorteile können in einer alternativen Ausführung der Erfindung auch dadurch erreicht werden, dass der für die Elektrochemie wichtige Abschnitt des Anschlussstifts im Inneren des Elektrolytkondensators eine Aluminiumbeschichtung eines Abschnitts aus einem anderen Material aufweist.

Zur zweckmäßigen Verarbeitung mit dem Glaslot im Versiegelungsabschnitt der Durchführung ist eine Ausgestaltung sinnvoll, bei der ein Stift aus Pt, Pt/Ir, FeNi, FeNiCo, FeCr, Nb, Ta, Mo, W, Cr, FeCr, V, Ti und weiteren Metallen oder deren Legierungen an den Aluminiumstift oder das Aluminiumblech angefügt, insbesondere angelötet, angeschweißt, angesteckt, angecrimpt oder angeklebt ist. Zur Erleichterung der weiteren Prozessführung kann insbesondere der außenseitige Anschlussstift einen sogenannten Nailhead aufweisen, abgeflacht, gekrümmt, mit einer angefügten Hülse oder Scheibe versehen oder sonst geeignet gestaltet sein. Der Nailhead erleichtert ein anschließendes Weichlöten des Außenanschlusses, speziell auch mittels eines Reflow-Verfahrens.

Ein weiterer bevorzugter Aufbau der vorgeschlagenen Durchführung zeichnet sich dadurch aus, dass der Anschlussstift im Aluminiumflansch von einem Keramikring, insbesondere aus Al₂O₃-Keramik, umgeben ist, so dass der Keramikring für den Glaslot-Pfropf beim Lötprozess einen Abschluss bildet und den Glasverlauf damit positiv beeinflusst, so dass der Glaslot-Pfropf bei der Herstellung der Durchführung mit einer höheren Ausbeute eine hermetische Dichtung erzielen kann. Eine solche kombinierte Glas-Keramik-Durchführung erhält durch die hochgradig steife Keramik-Komponente zusätzlich eine hohe mechanische Stabilität, und durch die Keramik-Komponente wird auch eine hochwertige und zuverlässige elektrische Isolationsstrecke an den benachbarten Oberflächen von (Aluminium-)Flansch und Anschlussstift realisiert. Insbesondere bietet sie dem Glaslot-Pfropf einen mechanischen Schutz gegenüber seitlichen Krafteinwirkungen (Biegekräften), die am Anschlussstift angreifen können.

In einer weiteren Ausführung der Erfindung ist innenseitig des Glaslot-Pfropfs ein den Anschlussstift umgebender Füllstoff oder eine Beschichtung vorgesehen. Speziell ist der Füllstoff oder die Beschichtung so dimensioniert und angeordnet, dass er oder sie eine Fügestelle im Anschlussstift maskiert, wenn die Fügestelle gegenüber dem Kondensatorinneren inkompatibel ist und durch den Glaslot-Pfropf nicht ausreichend gegenüber dem Kondensatorinneren getrennt gehalten werden kann. Wenn der Glaslot-Pfropf selbst zum Kondensatorinneren inkompatibel ist, dann bedeckt der Füllstoff oder die Beschichtung die dem Kondensatorinneren zugängliche Oberfläche des Glaslot-Pfropfs. Dieser Füllstoff bzw. diese Beschichtung sichert die für die Funktion des Kondensators wichtige Kompatibilität zwischen den in seinem Inneren vorliegenden Materialien, die durch die außenseitig zur Erreichung hermetischer Dichtheit zu benutzenden Materialien nicht gewährleistet wäre. Der Füllstoff kann ebenfalls als Pfropfen, aber auch als Belag, Beschichtung Bedampfung o. ä. vorliegen und sollte alle gegenüber den Innen-Materialien inkompatiblen Oberflächen bedecken.

Eine zweckmäßige Verfahrensführung zur Herstellung der Durchführung, die speziell den Einsatz von Anschlussstift-Materialien mit einem vom Glaslot erheblich differierenden thermischen Ausdehnungskoeffizienten erlaubt, zeichnet sich dadurch aus, dass während der Erzeugung des Glaslot-Pfropfs der Anschlussstift mittels einer Wärmesenke gekühlt wird. Dadurch, dass hierbei der Anschlussstift kühler bleibt als das Glas, lassen sich die ansonsten auftretenden thermischen Spannungen so weit kontrollieren, dass eine hermetisch dichte Verlötung gelingt. Der Wärmeeintrag in das Glaslot kann beispielsweise durch IR-Strahlung (etwa eines CO₂-Lasers oder induktive Wärmeeinkopplung über den umgebenden Flansch o. ä. erfolgen.

Von Vorteil ist es unter diesem Aspekt im Übrigen, die Glaslotmenge gering und die wirksame dichtende Grenzfläche zwischen Glaslot und Anschlussstift klein zu halten, um die absoluten Beträge der mechanischen Spannungen zwischen dem Glaslot-Pfropf und Pin aufgrund gegenseitiger großer Unterschiede in den thermischen Ausdehnungskoeffizienten gering zu halten.

Vorteile und Zweckmäßigkeiten der Erfindung ergeben sich im Übrigen aus der nachfolgenden Beschreibung von Ausführungsbeispielen und -aspekten anhand der Figuren. Von diesen zeigen:
- Fig. 1: eine Gesamtansicht eines (Elektrolyt-)Kondensators,
- Fig. 2 bis 4: Längsschnittdarstellungen von Durchführungen gemäß Ausführungsformen der Erfindung und
- Fig. 5 bis 11: Längsschnittdarstellungen von Durchführungen gemäß weiteren Ausfüh- rungen der Erfindung, mit zusätzlichem Füllstoff.

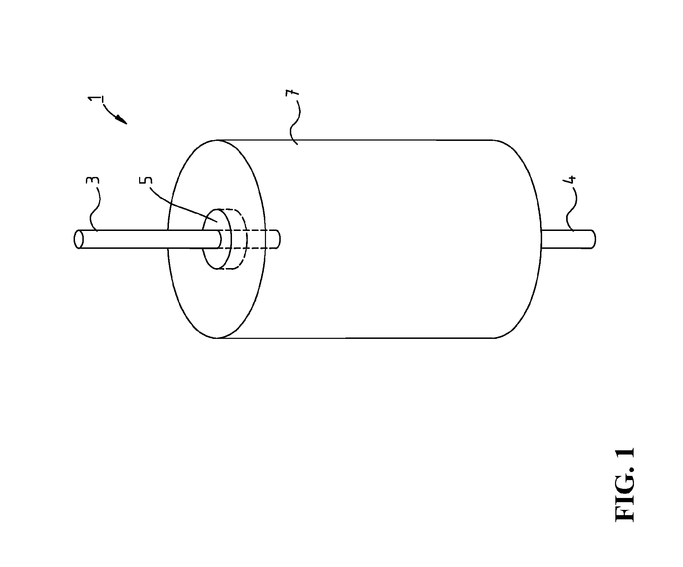

Fig. 1 zeigt in perspektivischer Darstellung eine bekannte Bauform eines Elektrolytkondensators 1 mit zwei Anschlussstiften 3 und 4, wobei skizzenartig gestrichelt eine Durchführung 5 an einer Stirnseite eines Gehäuses 7 dargestellt ist.

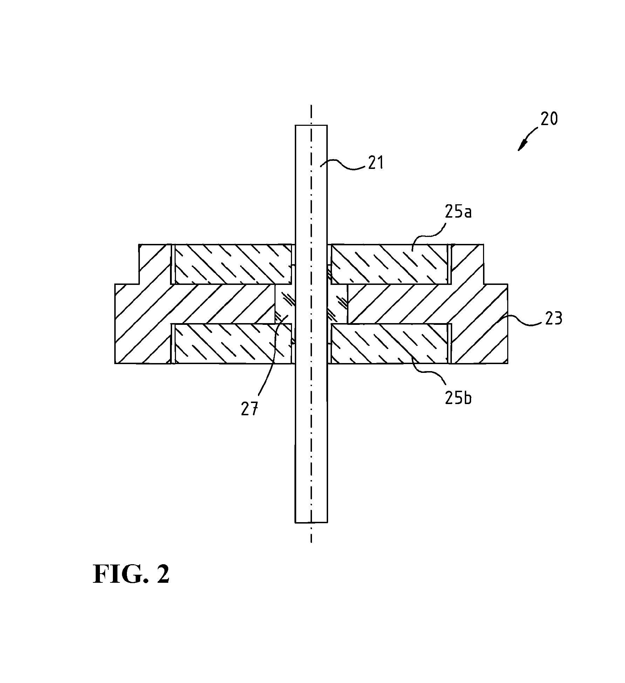

Fig. 2 zeigt als Ausführungsform der Erfindung eine Durchführung 20 mit einem Aluminium-Anschlussstift (Pin) 21, einem ober- und unterseitig mit flach zylindrischen Ausnehmungen versehenen Al-Flansch 23 und jeweils in dem Al-Flansch 23 liegenden Keramikscheiben 25a, 25b mit einer zentrischen Öffnung zum Durchgang des Al-Pins 21. Außenseitig des Al-Flansches 23 schließt sich an den Aufbau eine (hier nicht dargestellte) Gehäusewandung an, und zwischen dem Mittelteil des Al-Flansches 23 sowie den Innenwandungen der zentrischen Öffnungen der Keramikscheiben 25a, 25b sowie der Außenoberfläche des Pins 21 befindet sich ein Glaslot-Pfropf 27, der sowohl den Pin 21 als auch den Al-Flansch 23 hermetisch gegen die umgebenden Oberflächen abdichtet, so dass sich insgesamt eine hermetische Abdichtung zwischen dem Kondensatorinneren und -äußeren ergibt, die zudem mit einer hohen Ausbeute mechanisch stabil, elektrisch isolierend, maßhaltig, langzeitstabil und geometrisch kompakt ist.

Als Glaslot ist ein niedrigschmelzendes Glaslot mit einer Schmelztemperatur deutlich unterhalb derer des Aluminiums (660°C) einzusetzen, etwa ein bleihaltiges Lot des Typs G 017-052 der Firma Schott mit einer Löttemperatur von ca. 410°C oder ein bleifreies Lot des G 018-249, ebenfalls von Schott, mit einer Löttemperatur von 500°C. Spannungen aufgrund der voneinander abweichenden thermischen Ausdehnungskoeffizienten des Al-Pins und Al-Flansches einerseits und des Glaslots andererseits können weitestgehend durch die spezielle konstruktive Gestaltung vermieden werden, die den Einsatz einer sehr geringen Glasmenge zur Erzeugung des Glaslot-Pfropfes 27 beinhaltet. Des Weiteren ist in diesem Sinne eine Kühlung des Pins 21 über eine Wärmesenke als sinnvoll anzusehen.

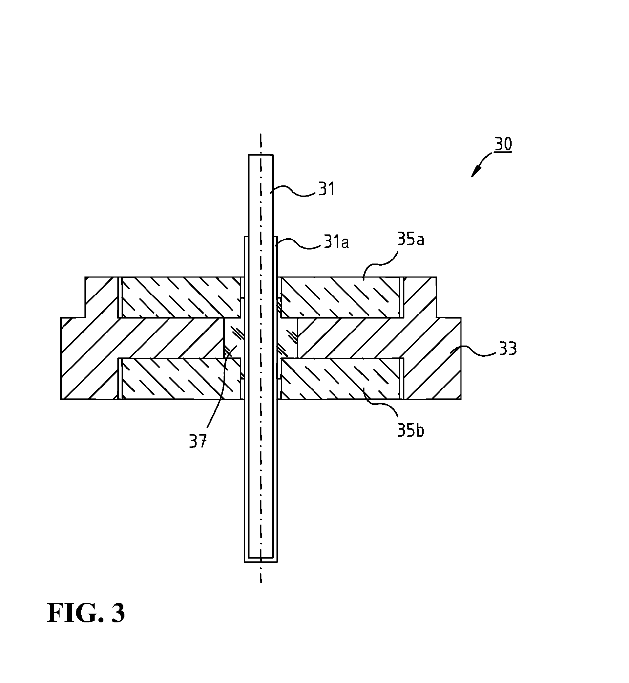

Fig. 3 zeigt einen der Ausführung nach Fig. 2 grundsätzlich ähnlichen Aufbau einer weiteren Durchführung 30. Gleiche oder ähnliche Teile sind mit an Fig. 2 angelehnten Bezugsziffern bezeichnet und werden hier nicht nochmals erläutert. Der wesentliche Unterschied gegenüber der Ausführung nach Fig. 2 besteht im Einsatz eines Pins 31 aus Platin. Dieser Pin ist mit einer Al-Beschichtung 31a versehen, die mindestens den kondensatorinneren Teil des Pins und den innenseitigen Abschnitt am Glas-Pfropf 37 umfasst. Die Beschichtung kann aber auch - wie in Fig. 3 angedeutet - bis in den kondensatoräußeren Bereich des Pins reichen, um beispielsweise die elektrochemischen Prozesse im Glaslot, die während der Hochtemperaturverlötung eine Rolle spielen, nicht zu beeinträchtigen. Mit diesem Aufbau des Anschlussstiftes ist dieser einerseits besser an den thermischen Ausdehnungskoeffizienten des Glases angepasst, und andererseits wird das Freiliegen von mit den übrigen Materialien im Inneren des Kondensators inkompatiblen Oberflächen durch die Al-Beschichtung vermieden. Die Al-Beschichtung kann im Kondensator mit einer adäquaten Oxidschicht versehen werden, und von außen ist der Pin löt-, schweiß-, ansteck-, ancrimp- oder elektrisch leitfähig anklebbar.

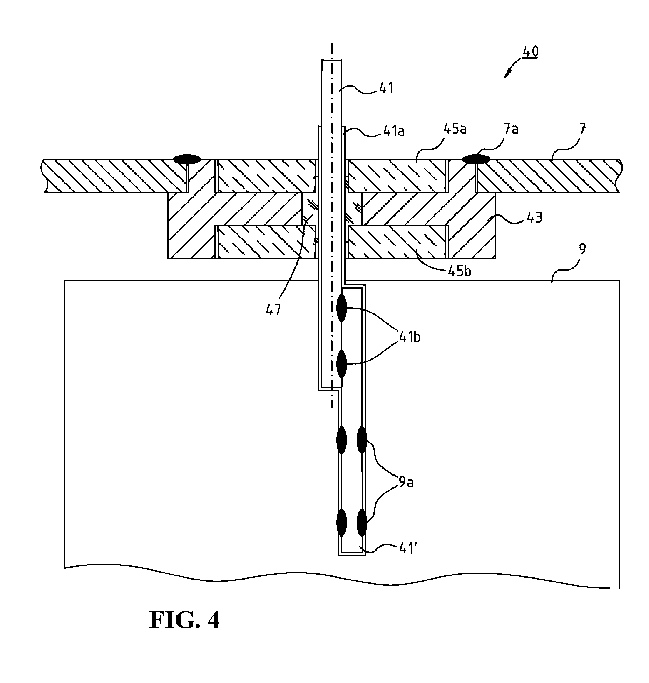

Fig. 4 zeigt als Fortbildung der in Fig. 3 gezeigten Ausführung eine weitere Durchführung 40, wo wiederum an Fig. 2 bzw. 3 angelehnte Bezugsziffern verwendet sind. Neben der eigentlichen Durchführung sind in dieser Darstellung auch die angrenzenden Abschnitte des Gehäuses 7 gezeigt, die über eine Schweißnaht 7a mit dem Al-Flansch 43 gasdicht verbunden sind. Des Weiteren zeigt die Figur ein Al-Kondensatorblech 9, das eine wesentliche Funktionskomponente des Kondensatoraufbaus bildet, sowie Füge- bzw. Schweißpunkte 9a zu dessen Verbindung mit dem Anschlussstift.

Eine weitere Besonderheit des Aufbaus nach Fig. 4 besteht darin, dass der Anschlussstift hier aus einem äußeren Stift 41 aus einem anderen Material als Al, z. B. Pt, Pt/Ir, FeNi, FeNiCo, FeCr, Nb, Ta, Mo, W, Cr, FeCr, V oder Ti und einem inneren Stift 41' aus Al aufgebaut ist, die über Schweißpunkte 41b miteinander verbunden sind. Die gesamte kondensatorinnere Oberfläche beider Teil-Anschlussstifte 41, 41' sowie die Oberfläche der Schweißpunkte 41b ist mit einer mit dem Kondensatorinneren kompatiblen Al-Beschichtung 41a bedeckt. In dieser Figur ist zu erkennen, dass alle dem Inneren des Kondensators zugewandten Oberflächen, nämlich diejenigen des Gehäuses 7, des Flansches 43, der Keramikscheibe 45b und des inneren Abschnittes des Anschlussstifts 41/41', aus Aluminium oder einem hierzu kompatiblen Metall oder einer kompatiblen Keramik bestehen. Die Al-Beschichtung 41a, die sich über dem äußeren Stift 41, dem inneren Stift 41', den Fügestellen 41b und 9a und eventuell auch über dem Al-Kondensatorblech 9 befindet, kann im Kondensator mit einer (nicht dargestellten) adäquaten Oxidschicht versehen sein, welche dann ein funktionsfähiges Dielektrikum des Kondensators bildet.

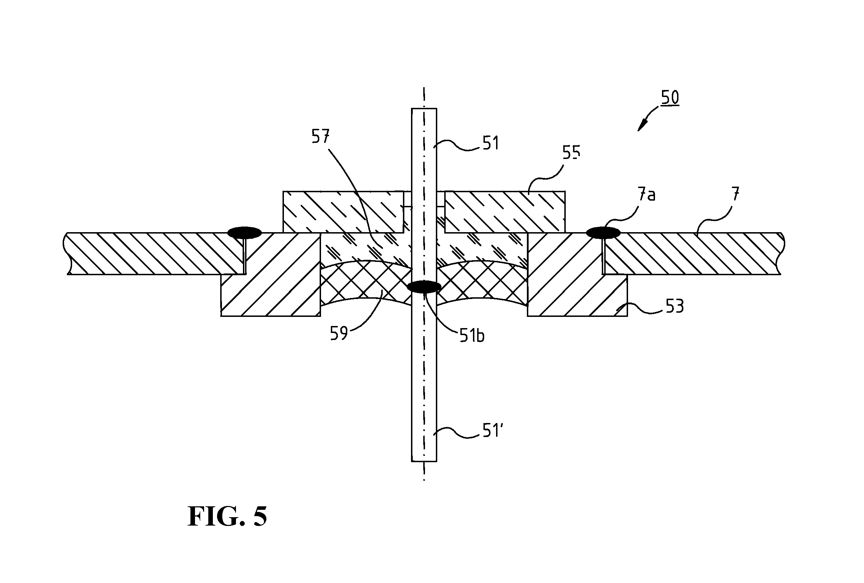

Fig. 5 zeigt eine weitere erfindungsgemäße Durchführung 50 mit einem zusammengesetzten Anschlussstift 51/51' aus einem ersten Stiftabschnitt 51 aus Pt, Pt/Ir, FeNi, FeNiCo, FeCr, Nb, Ta, Mo, W, Cr, FeCr, V oder Ti oder einer Legierung aus diesen oder einem ähnlichen Material und einem zweiten Stiftabschnitt 51' aus Aluminium. Beide Abschnitte sind durch eine Fügestelle 51b, die durch Schweißen, Löten, crimpen, elektrisch leitfähiges Kleben oder ein anderes, geeignetes Verfahren realisiert ist, miteinander verbunden.

Weitere Unterschiede zu den vorhergehenden Ausführungen bestehen im Vorsehen eines konstruktiv einfacher aufgebauten Al-Flansches 53 und einer einzigen Keramikscheibe 55, welche hier plan auf der Oberseite des Flansches 53 aufliegt. Die Verwendung eines Stiftabschnitts 51, dessen Wärmeausdehnungskoeffizient an den Glaspfropfs 57 angepasst oder kleiner ist, führt dazu, dass die gemeinsame Dichtlänge größer ausfallen darf, als bei Verwendung eines Stifts aus Al wie in den Ausführungen der Fig. 2 bis Fig. 4, somit das Glas voluminöser ausgeführt sein darf und damit eine höhere mechanische Stabilität des Aufbaus erreicht wird. Seine dem Inneren des Kondensators zugewandte Oberfläche sowie das aus dieser geringfügig hervorstehende Ende des äußeren Stiftabschnitts 51 sowie die Fügestelle 51b sind in einen Füllstoff-Pfropf 59 eingebettet. Beim Füllstoff handelt es sich etwa um Gummi, eine Silikonmasse oder einen halogen- bzw. chlorfreien Kunststoff. Der Füllstoff-Pfropf 59 sichert, dass keinerlei mit den im Inneren des Kondensators typischerweise verwendeten Materialien inkompatible Materialien der Durchführungskonstruktion zum Kondensatorinneren hin offen liegen.

In einer Designvariante der Fig. 5 wird mindestens die Fügestelle und der sich daran anschließende Teil des Stifts und der Übergang zur frei liegenden Oberfläche des Glaspfropfs von einem Füllstoff-Pfropf überdeckt und damit das Kondensatorinnere vor inkompatiblen Bestandteilen geschützt. Der Füllstoff-Pfropf kann dabei auch als vergleichsweise dünne, Volumen sparende Beschichtung ausgeführt sein, die einen unzulässigen Ionenaustausch der inkompatiblen Bestandteile mit dem Kondensatorinneren unterbindet. Wenn der Glaspfropf gegenüber dem Kondensatorinneren aus einem kompatiblen Material ausgeführt ist, dann muss er von dem Füllstoff-Pfropf bzw. der Beschichtung nicht vollständig bedeckt sein.

In einer weiteren Designvariante der Fig. 5 kann die Fügestelle und zusätzlich ein Teil des Al-Stiftabschnitts vom Glaspfropf teilweise oder vollständig umschlossen sein. Selbst bei einem gleichzeitigen Umschließen der Fügestelle und dem sich daran anschließenden Teil des Al-Stiftabschnitts durch den Glaspfropf kann der Glaspfropf aufgrund der vergleichsweise großen Wärmeausdehnungskoeffizienten der Fügestelle und des Stiftes im Allgemeinen keinen hermetisch dichten Verschluss dieser Teile gegenüber dem Kondensatorinneren sicherstellen. Diese Aufgabe sollte in einer bevorzugten Designvariante durch einen Füllstoff-Pfropf oder von einer entsprechenden Beschichtung übernommen werden, die mindestens den Übergang vom Glaspfropf zum Stift bzw. zur Fügestelle vollständig bedeckt und damit gegenüber dem Kondensatorinneren maskiert.

In einer weiteren Designvariante der Fig. 5 umschließen zwei Keramik-Ringe außen und innen den Glaspfropf, wobei der zweiteilige Stift durch eine Öffnung der Keramikringe geführt ist und vom Glaspfropf hermetisch dicht umschlossen wird. Die Fügestelle kann sich dabei ganz oder teilweise innerhalb des Glaspfropfs und ganz oder teilweise innerhalb des im Kondensatorinneren befindlichen Keramikrings befinden. In einer bevorzugten Designvariante werden mindestens die gegenüber dem Kondensatorinneren inkompatiblen Bestandteile, also die Fügestelle der Stiftabschnitt und bei der Wahl eines inkompatiblen Glaspfropf-Werkstoffs auch der Glaspfropf, von einem Füllstoff-Pfropf bzw. einer Beschichtung überdeckt. Dabei kann der innere Keramikring und der Flansch, sowie das Gehäuse ganz oder teilweise vom dem Füllstoff-Pfropf bzw. der Beschichtung überdeckt bzw. benetzt sein.

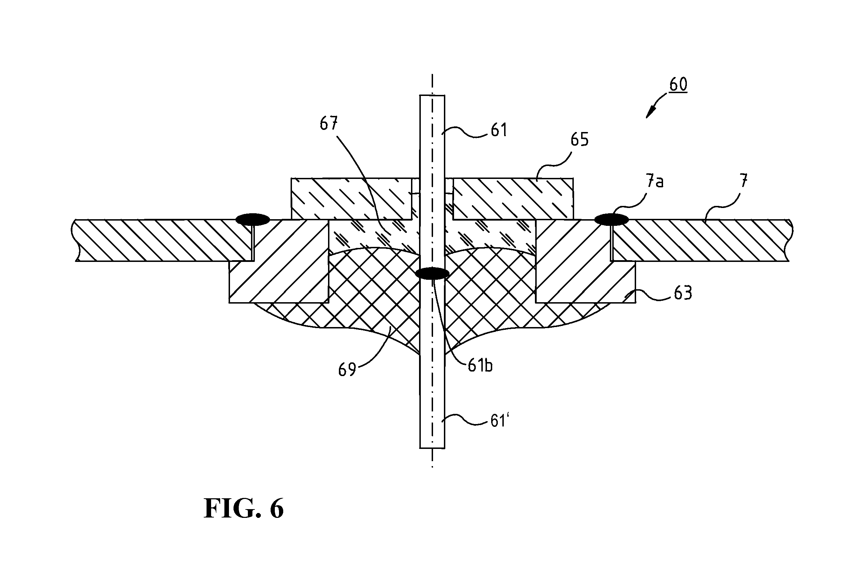

Fig. 6 zeigt eine geringfügige Abwandlung der in Fig. 5 dargestellten Konstruktion 60, bei der der Füllstoff-Pfropf 69 die Bohrung des Flansches 53 vollständig ausfüllt und einen größeren Bereich des inneren Anschlussstift-Abschnitts 61' benetzt. Dies hat den Vorteil, dass sich die Fügestelle 61b zwischen den Stiftabschnitten 61 und 61' nicht innerhalb der Bohrung des Flansches 63 befinden muss und daher leichter gebildet werden kann.

Die Fügestelle kann im Übrigen auch eine Schweiß- oder Löt- oder elektrisch leitfähige Klebeverbindung sein, oder die Stiftabschnitte können (über ein zusätzliches Element oder über einer Öffnung (Bohrung) oder eines Spalts der beiden Stifte) miteinander vercrimpt sein.

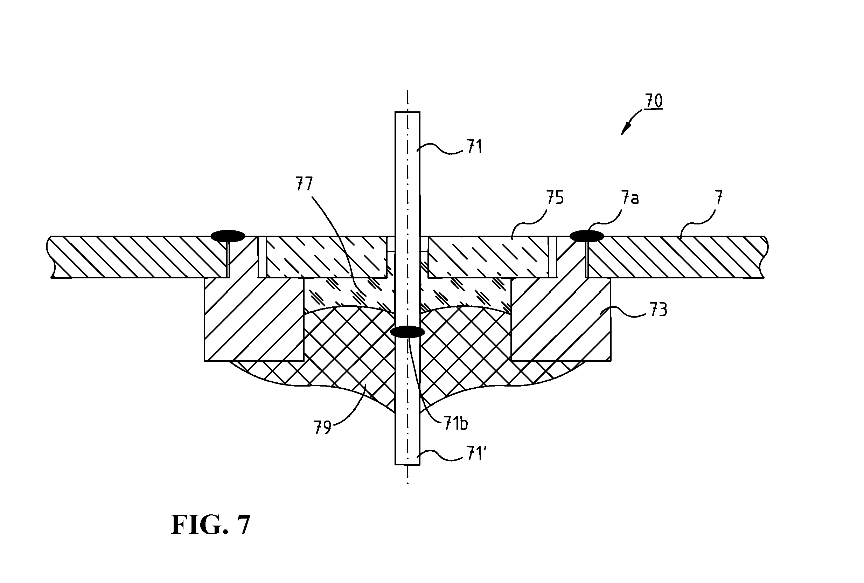

Eine weitere, in Fig. 7 gezeigte Durchführung 70 stellt eine geringfügige Abwandlung der in Fig. 6 gezeigten Ausführung dar, bei der in der Oberseite des Flansches 73 eine erweiterte Ausnehmung vorgesehen und in diese die Keramikscheibe 75 derart eingefügt ist, dass ihre obere Stirnfläche in etwa bündig mit der oberen Stirnfläche des Flansches 73 abschließt und somit insgesamt (unter Einbeziehung des oberen Gehäuseabschnitts 7) eine im Wesentlichen ebene Oberfläche der Durchführung und der zugehörigen Kondensator-Stirnfläche geschaffen wird. In einer hier nicht gezeigten Designvariante kann dieses Prinzip mit der eingelassenen Keramikscheibe auch auf eine auf der Innenseite des Kondensators befindlichen Keramikscheibe übertragen sein. Die mit einer zentralen Öffnung versehene(n) Keramikscheibe(n) übernimmt (übernehmen) dabei die Aufgabe der Zentrierung des Stiftes gegenüber der zentralen Flanschöffnung während der Verlötung der Glas/Keramik-Durchführung im Herstellungsprozess.

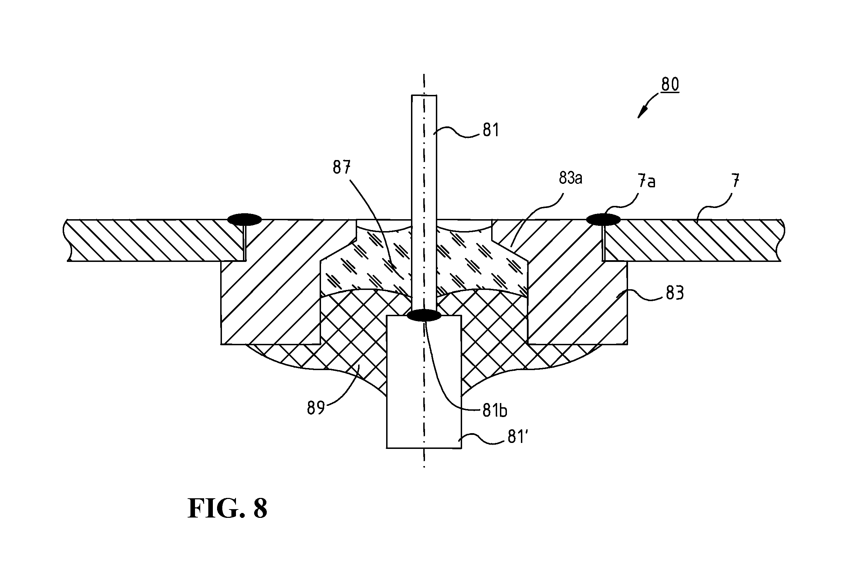

Bei der in Fig. 8 gezeigten weiteren Durchführung 80 hat der Flansch 83 eine weiter modifizierte Gestalt, die einen Fortfall der bei den anderen Ausführungen vorgesehenen Keramikscheibe(n) ermöglicht. Um für einen günstigen Glaslotverlauf und eine günstige Position des Glaslot-Pfropfens 87 im Flansch 83 zu sorgen, hat dieser hier einen nach innen weisenden angeschrägten Abschnitt 83a, der während des Herstellprozesses den Glaslot-Pfropf positioniert und in der Anwendung bis zu einem gewissen Grade die mechanische Funktion der Keramikscheibe übernimmt. Der innere Abschnitt 81' des Anschlussstiftes ist hier als Designvariante breiter (bzw. dicker) als der äußere Stiftabschnitt 81, in dessen Bereich die hermetisch dichte Versiegelung über das Glaslot hergestellt wird. Es kann sich bei dem inneren Abschnitt übrigens auch um ein Blech verschiedener Gestalt o. ä. handeln.

Der angeschrägte Abschnitt 83a kann in einer weiteren Designvariante wegfallen und der Flansch somit eine stufenlose, zylinderförmige Öffnung besitzen, wobei dann durch entsprechende Maßnahmen während des Herstellungsprozesses, wie z. B. den Einsatz von vom Glaslot-Pfropf nicht benetzbaren Zentrierungen wie Graphit, die Position des Glaslot-Pfropfes in der Öffnung des Flansches festgelegt wird.

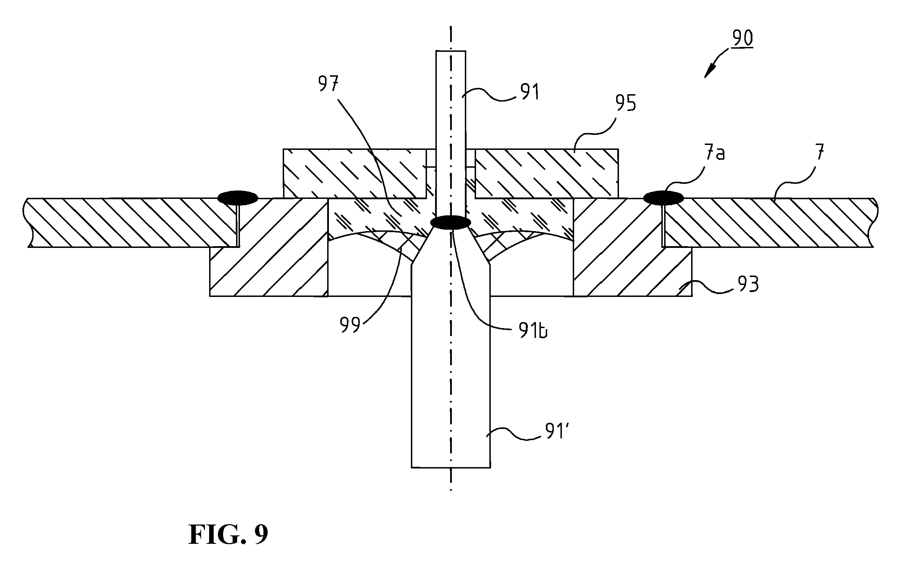

Fig. 9 zeigt eine weitere Ausführung einer Durchführung 90, bei der die Keramikscheibe 95 flach auf der Oberfläche des Flansches 93 angebracht ist. Der innere Abschnitt 91' des Anschlussstiftes ist breiter (und/oder dicker) als der äußere Stiftabschnitt 91 aufgebaut. Zusätzlich weist der innere Abschnitt 91' einen zur Fügestelle 91b hin abgeschrägten Abschnitt auf. Wie schon in den anderen Ausführungen gezeigt, liegt die Fügestelle 91b auch hier innerhalb des Glaslot-Pfropfens 97, der die Fügestelle 91b komplett umschließt. Zusätzlich bedeckt ein Füllstoffpfropf die Umgebung in der Nähr und/oder um die Fügestelle 91b, wobei jedoch die Flanschöffnung des Flansches 93 nicht komplett vom Füllstoffpfropf ausgefüllt ist. Der Glaslot-Pfropfen 97 und/oder der Füllstoff-Pfropf 99 kann aus einem zum Kondensatorinneren kompatiblen Material geformt sein, um das Kondensatorinnere (beispielsweise das Elektrolyt) vor nicht kompatiblen Bauteilen außerhalb des Kondensators zu schützen.

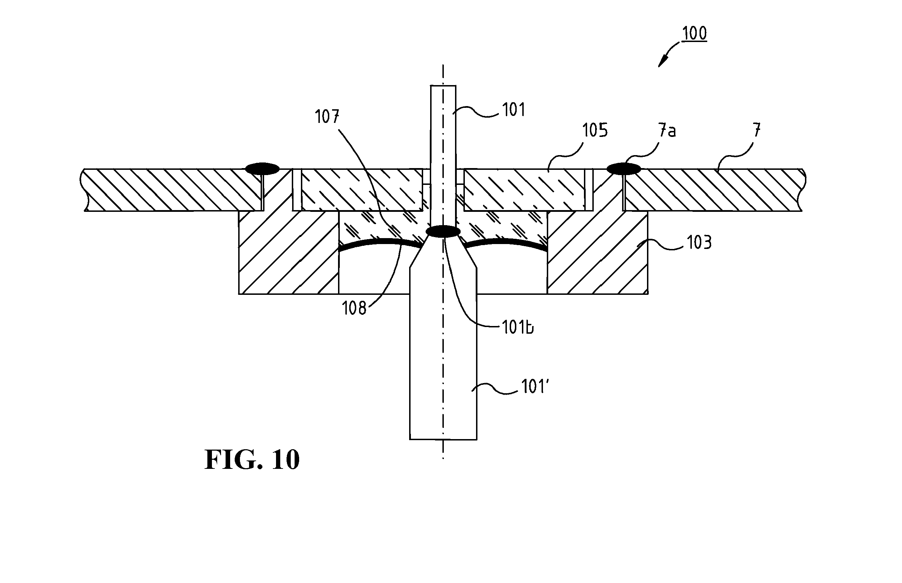

Fig. 10 zeigt eine Alternative zur Ausführungsform aus Fig. 9. Dort ist eine Durchführung 100 gezeigt, welche auf der Oberfläche des Flansches 103 eine Aussparung aufweist, in welche die Keramikscheibe 105 eingesetzt ist. Die Durchführung 100 ist dann so geformt, dass die Außenoberfläche der Keramikscheibe 105 mit der Oberfläche des Flansches 103 bündig liegen. Dadurch wird eine weitgehende flache und einheitliche Außenoberfläche der Durchführung 100 und des anschließenden oberen Kondensatorgehäuses 7 gewährleistet. Der innere Abschnitt 101' des Anschlussstiftes ist breiter (und/oder dicker) als der äußere Stiftabschnitt 101 aufgebaut. Zusätzlich weist der innere Abschnitt 101' einen zur Fügestelle 101b hin abgeschrägten Abschnitt auf. Wie schon in den anderen Ausführungen gezeigt, liegt die Fügestelle 101b auch hier innerhalb des Glaslot-Pfropfens 107, der die Fügestelle 101b komplett umschließt. Der Füllstoff-Pfropf ist durch eine dünne Beschichtung 108 ersetzt, welche den Glaslot-Pfropfen 107 komplett bedeckt. Der Füllstoff-Pfropf 107 kann aus einem zum Kondensatorinneren kompatiblen Material geformt sein, um das Kondensatorinnere (beispielsweise das Elektrolyt) vor nicht kompatiblen Bauteilen außerhalb des Kondensators zu schützen. So wird innerhalb des Kondensators Platz eingespart und es wird unzulässiger Ionenaustausch mit nicht kompatiblen Komponente mit dem Kondensatorinneren vermieden. Die Füllstoff-Schicht kann beispielsweise Gummi, eine Silikonmasse einen halogen- bzw. chlorfreien Kunststoff oder Polymere wie Epoxidharz, Polyimid (beispielsweise Kapton®) oder Perylene® sein.

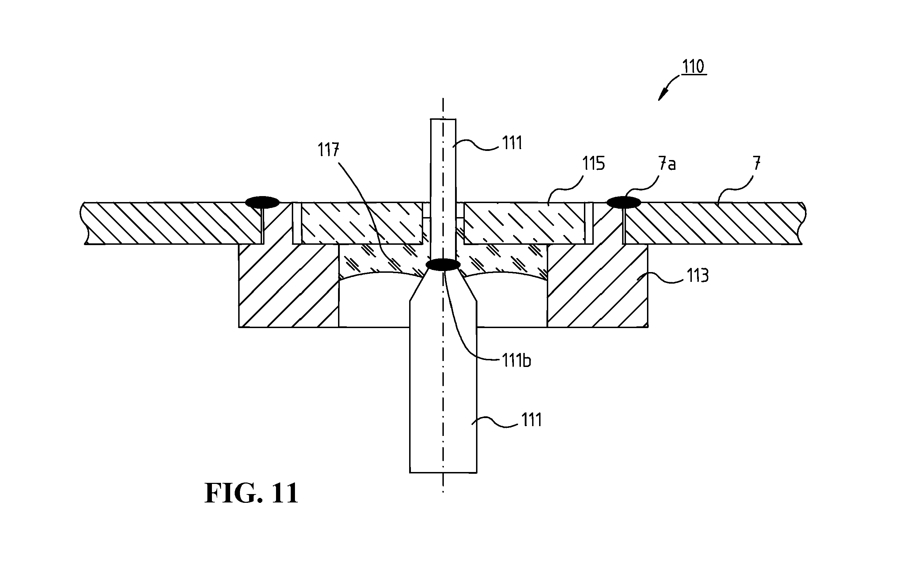

Fig. 11 zeigt eine weitere Alternative der Durchführung en, die auf den Ausführungsformen der Fig. 9 oder 10 basiert. In dieser Durchführung 110 wurde auf die Füllstoffbeschichtung 108 bzw. den Füllstoff-Pfropf 99 komplett verzichtet. Der Glaslot-Pfropfen 117 ist dabei aus einem zum Kondensatorinneren kompatiblen Material geformt. So kann auf den Füllstoff in Form entweder eines Pfropfs oder einer Beschichtung verzichtet und trotzdem das Kondensatorinnere (beispielsweise das Elektrolyt) vor nicht kompatiblen Bauteilen außerhalb des Kondensators geschützt werden. Auch hier umschließt der Glaslot-Propfen 117 die Fügestelle 111b, mit Hilfe derer der innere Abschnitt 111' und der äußere Abschnitt 111 des Anschlussstiftes miteinander elektrisch verbunden ist. Durch die komplette Umschließung der Fügestelle 117 wird sie vor dem Medium im Kondensatorinneren geschützt.

Die Ausführung der Erfindung ist nicht auf die oben beschriebenen Beispiele und hervorgehobenen Aspekte beschränkt, sondern ebenso für verschiedene, weitere Kondensatortypen und in zahlreichen Abwandlungen möglich, die im Rahmen fachgemäßen Handelns liegen.

## Patentansprüche

1. Durchführung eines Kondensator, insbesondere Elektrolyt-, Tantal- oder Niob-Kondensators, insbesondere in einem medizinelektronischen Implantat, mit einem Anschlussstift, der mindestens im Inneren des Kondensators einen zum Kondensatorinneren kompatiblen Abschnitt aufweist, einem den Anschlussstift umgebenden Aluminium-, Titan-, Tantal, oder Niob-Flansch und einem den Anschlussstift gegenüber dem Flansch hermetisch abdichtenden Glaslot-Pfropf.

2. Durchführung nach Anspruch 1, wobei der Anschlussstift in dem Bereich seiner Länge, in dem er in Kontakt mit dem Glaslot-Pfropf steht, mindestens einen Kern aus einem Material aufweist, das annähernd den gleichen oder einen geringeren thermischen Ausdehnungskoeffizienten wie das Glaslot hat.

3. Durchführung nach Anspruch 2, wobei das Material des Anschlussstifts oder seines Kerns in dem Bereich seiner Länge, in dem er direkt oder über eine Beschichtung in Kontakt mit dem Glaslot-Pfropf steht, Pt, Pt/Ir, FeNi, FeNiCo, FeCr, Nb, Ta, Mo, W, Cr, FeCr, V, Ti oder eine Legierung mit Metallen dieser Gruppe ist.

4. Durchführung nach einem der vorangehenden Ansprüche, wobei der zum Kondensatorinneren kompatible Abschnitt des Anschlussstifts im Inneren des Kondensators ein Aluminiumstift, ein Aluminiumblech oder eine Aluminiumfolie ist.

5. Durchführung nach Anspruch 4, wobei der Aluminiumstift, das Aluminiumblech oder die Aluminiumfolie an einen Abschnitt des Anschlussstifts aus einem anderen Material angefügt, insbesondere angelötet, angeschweißt, angecrimpt oder elektrisch leitfähig angeklebt, ist.

6. Durchführung nach einem der Ansprüche 1 bis 3, wobei der zum Kondensatorinneren kompatible Abschnitt des Anschlussstifts im Inneren des Elektrolytkondensators eine Aluminiumbeschichtung eines Abschnitts aus einem anderen Material aufweist.

7. Durchführung nach einem der Ansprüche 1 bis 3, wobei der Abschnitt des Anschlussstifts im Außenbereich des Kondensators ein Stift oder ein Blech aus weichlötbarem Material wie Nickel, Eisen, Kupfer, Palladium, Gold oder Silber oder einer Legierung mit einem dieser Metalle ist.

8. Durchführung nach Anspruch 7, wobei ein Aluminium, Tantal-, Niob- oder Titanstift an den weichlötbaren Stift oder das Blech angefügt, insbesondere angelötet, angeschweißt, angecrimpt oder elektrisch leitfähig angeklebt oder mit diesem über den Glaslot-Pfropf verbunden ist.

9. Durchführung nach Anspruch 7 oder 8, wobei der außenseitige Nickel-, Eisen-, Kupfer-, Palladium-, Gold-, Silber- oder Legierungsstift einen Nailhead, eine Hülse, eine Abflachung oder Funktionskrümmung aufweist.

10. Durchführung nach einem der vorangehenden Ansprüche, wobei der Anschlussstift im Aluminiumflansch von mindestens einem Keramikring, insbesondere aus Al₂O₃-Keramik, umgeben und der Glaslotpfropf so ausgeführt ist, dass er einen Ringspalt zwischen dem Anschlussstift und dem Keramikring hermetisch dicht verschließt.

11. Durchführung nach einem der vorangehenden Ansprüche, wobei innenseitig des Glaslot-Pfropfs ein den Anschlussstift umgebender Füllstoff oder eine Beschichtung vorgesehen ist.

12. Durchführung nach Anspruch 11, wobei der Füllstoff oder die Beschichtung so dimensioniert und angeordnet ist, dass er/sie eine Fügestelle im Anschlussstift maskiert.

13. Elektrolytkondensator mit einer Durchführung nach einem der vorangehenden Ansprüche.

14. Verfahren zur Herstellung einer Durchführung nach einem der Ansprüche 1 bis 12, wobei während der Erzeugung des Glaslot-Pfropfs der Anschlussstift mittels einer Wärmesenke gekühlt wird.

15. Verwendung einer Durchführung nach einem der Ansprüche 1 bis 12, wobei ein außenseitiges Weichlöten am Anschlussstift mittels eines Reflow-Verfahrens ausgeführt wird.
